# FASCICULE DE BREVET EUROPEEN

(11) **EP 1 199 994 B1**
(45) Date de publication et mention de la délivrance du brevet: **18.10.2006**
(21) Numéro de dépôt: 00949615.9
(22) Date de dépôt: 03.07.2000
(51) Int. Cl.: A61B 17/70

(54) **PLAQUE D'OSTEOSYNTHESE VERTEBRALE ET SYSTEME D'OSTEOSYNTHESE**
PLATTE UND SYSTEM FÜR DIE WIRBELSÄULENOSTEOSYNTHESE
VERTEBRAL OSTEOSYNTHESIS PLATE AND OSTEOSYNTHESIS SYSTEM

(30) Priorité: 01.07.1999 FR 9908495
(43) Date de publication de la demande: 02.05.2002
(73) Titulaire: Spinevision S.A., 75012 Paris (FR)
(72) Inventeur: BOLGER, Ciaran, Bristol BS36 2NE (GB); BOLGER, John, Dublin 16 (IE)
(74) Mandataire: Breese, Pierre
(86) Numéro de dépôt international: PCT/FR2000/001893
(87) Numéro de publication internationale: WO 2001/001874

(56) Documents cités:
- EP-A- 0 556 548
- EP-A- 0 705 572
- DE-A- 4 201 043

## Description

La présente invention concerne le domaine de la chirurgie d'ostéosynthèse rachidienne. Elle concerne plus particulièrement une plaque d'ostéosynthèse vertébrale destinée à immobiliser l'une par rapport à l'autre au moins deux vertèbres adjacentes.

De telles plaques sont habituellement découpées dans un matériau biocompatible, notamment métallique, par exemple du titane, rigide ou flexible. Elles présentent des lumières circulaires ou oblongues pour le passage des vis de fixation ou des implants vertébraux formés par un organe fileté.

À titre d'exemple, les demandes de brevets français FR 2763828 ou FR 2766353 décrivent des systèmes d'ostéosynthèse vertébrale mettant en oeuvre des plaques connues dans l'état de la technique.

On connaît également la demande de brevet français FR 2737402 qui décrit un matériel de stabilisation antérieure du rachis cervical destiné à fixer les vertèbres et restaurer un espace intersomatique normal. Le système comprend une plaque rectangulaire à double courbure, pourvue de lumières ovoïdes, des vis d'ancrage corporéales, et des bouchons filetés de condamnation venant se loger dans le taraudage aménagé dans la partie supérieure de la vis d'ancrage.

Ces plaques d'ostéosynthèse de l'art antérieur sont difficiles à poser. En effet, le chirurgien commence par corriger l'alignement du rachis avec un instrument de correction temporaire présentant des tiges de correction destinées à être fixées dans les vertèbres qui gênent la pose de la plaque et sa fixation. Si l'instrument de correction est retiré avant fixation définitive de la plaque, il est nécessaire de procéder à des ajustements de l'alignement de la plaque avant la fixation définitive.

Pour remédier à cet inconvénient, il a été proposé, dans la demande de brevet allemand N° DE 4 201 043 une plaque d'ostéosynthèse vertébrale de forme générale rectangulaire présentant au moins quatre lumières pour le passage d'un moyen de fixation ainsi que deux fentes longitudinales s'ouvrant sur un bord transversal de la plaque, pour permettre une mise en place et une fixation définitive de la plaque sans retrait préalable de l'instrument de correction de l'orientation relative des vertèbres.

L'inconvénient majeur de cette plaque réside dans le fait que le chirurgien est obligé de réaliser six trous : deux trous pour permettre la mise en oeuvre de l'instrument de correction de l'orientation relative des vertèbres et quatre trous pour la fixation de la plaque d'ostéosynthèse vertébrale.

En outre, la présence des fentes longitudinales oblige à augmenter la largeur de la plaque d'ostéosynthèse.

Le but de la présente invention est de proposer une plaque évitant ces inconvénients. Pour ce faire, la présente invention se rapporte à une plaque d'ostéosynthèse vertébrale pour le maintien de la correction de l'orientation relative des vertèbres à l'aide d'un instrument de correction, ladite plaque d'ostéosynthèse étant de forme générale rectangulaire et présentant au moins quatre lumières pour le passage, dans chacune, d'un moyen de fixation. La plaque d'ostéosynthèse selon l'invention est remarquable, dans son acception la plus large, en ce qu'au moins deux lumières s'ouvrent sur un bord latéral de ladite plaque d'ostéosynthèse pour permettre une mise en place et une fixation de la plaque sans retrait préalable de l'instrument de correction de l'orientation relative des vertèbres.

Le bord latéral de la plaque s'entend d'un bord situé dans la même direction que la direction longitudinale de la plaque, la direction longitudinale de la plaque étant sensiblement confondue avec la direction de la colonne vertébrale, là où est opérée l'ostéosynthèse. Le bord latéral comprend également le coin situé à l'angle avec le bord transversal.

Ainsi, lorsque le chirurgien a corrigé l'orientation relative des vertèbres à l'aide des tiges de correction de l'instrument de correction implantées dans les vertèbres, il est en mesure de positionner la plaque d'ostéosynthèse de telle sorte que les tiges de correction de l'instrument de correction passent dans lesdits trous débouchant et de commencer à fixer la plaque, tout en laissant les tiges de correction en position.

Avantageusement, les trous réalisés pour la fixation des tiges de correction de l'instrument de correction de l'orientation relative des vertèbres servent également, après fixation partielle de la plaque d'ostéosynthèse et retrait des tiges de correction de l'instrument de correction, à la fixation complète de la plaque d'ostéosynthèse.

Avantageusement, l'une au moins des lumières débouchant sur le bord latéral de la plaque présente une forme coudée, en "L" ou autre, comprenant un premier segment transversal s'ouvrant sur le bord latéral de la plaque prolongé par un deuxième segment longitudinal orienté selon une direction sensiblement longitudinale.

La plaque selon l'invention participe ainsi activement, dès son introduction entre les tiges de correction de l'instrument, au maintien des éléments du rachis.

La plaque peut donc présenter deux lumières débouchantes en forme de « L », ou une lumière débouchante en forme de « L » et une deuxième lumière débouchante orientée transversalement, ou deux lumières débouchantes orientées transversalement.

La plaque d'ostéosynthèse vertébrale présente de préférence deux lumières débouchantes de forme coudée, en "L" ou autre, comprenant un premier segment transversal s'ouvrant sur le bord de la plaque prolongé par un deuxième segment longitudinal orienté selon une direction sensiblement longitudinale.

Avantageusement également, l'une au moins des lumières ne débouchant pas sur le bord latéral de la plaque présente un deuxième segment orienté selon une direction sensiblement longitudinale, afin de permettre la compensation post-opératoire automatique des effets du tassement de l'espace intervertébral occupé, le plus souvent, par un greffon osseux.

De préférence, l'espacement des lumières s'ouvrant sur le bord de la plaque correspond à la distance intersomatique entre N vertèbres, N étant un nombre entier supérieur ou égal à deux.

Selon une variante, la plaque comporte en outre au moins une lumière prévue sensiblement sur l'axe longitudinal médian pour la fixation d'un greffon osseux ou autre élément intersomatique.

Selon une variante préférée, la plaque comporte en outre au moins un trou de fixation d'une plaque de couverture destinée à couvrir au moins partiellement lesdits moyens de fixation, après fixation, ladite plaque de couverture étant, de préférence cintrée et comportant également de préférence au moins un trou de fixation, voire deux trous de fixation dont l'un au moins est oblongue afin de permettre par serrage de la plaque de couverture contre la plaque d'ostéosynthèse de l'adapter au profil de la plaque d'ostéosynthèse.

Selon un mode de réalisation particulier de l'invention, l'une au moins desdites lumières pour le passage d'un moyen de fixation, présente au moins deux positions d'accueil d'un moyen de fixation : une position d'accueil en surface dans laquelle ledit moyen de fixation est sensiblement oblique selon un angle fixe par rapport à ladite plaque, et une position d'accueil en profondeur dans laquelle ledit moyen de fixation présente une position oblique selon un angle variable.

L'invention concerne également un système d'ostéosynthèse vertébrale comprenant une pluralité de plaques d'ostéosynthèse vertébrale pour le maintien de la correction de l'orientation relative des vertèbres réalisée à l'aide d'un instrument de correction, lesdites plaques d'ostéosynthèse étant de forme générale rectangulaire et présentant chacune au moins quatre lumières pour le passage, dans chacune des lumières, d'un moyen de fixation, au moins deux lumières s'ouvrant sur un bord latéral de ladite plaque d'ostéosynthèse pour permettre une mise en place et une fixation de la plaque sans retrait préalable de l'instrument de correction de l'orientation relative des vertèbres, l'espacement des lumières étant spécifique à chaque plaque pour permettre la sélection par le chirurgien de la plaque présentant un espacement des lumières proche de la distance intervertébrale des vertèbres du patient en cours d'opération.

L'invention sera mieux comprise à la lecture de la description faite ci-après, à titre purement explicatif, d'un mode de réalisation de l'invention, en référence aux figures annexées :
- la figure 1 représente une vue schématique d'une plaque d'ostéosynthèse vertébrale selon l'invention ;
- la figure 2 représente une vue en perspective de la plaque d'ostéosynthèse vertébrale en cours de fixation, sans les vertèbres ;
- la figure 3 représente une vue en perspective de la plaque d'ostéosynthèse vertébrale de la figure 2 munie d'une plaque de couverture ;
- la figure 4 représente une vue de dessus, partielle, d'une lumière à deux positions d'accueil de la plaque d'ostéosynthèse vertébrale selon l'invention ;
- les figures 5 et 6 représentent respectivement une vue de dessus, partielle, des deux possibilités de positionnement en surface ou en profondeur, des vis de fixation osseuse dans une lumière de la plaque d'ostéosynthèse vertébrale selon l'invention ;
- les figures 7 et 8 représentent respectivement une vue de face des deux vis de fixation qui peuvent être positionnées en surface ou en profondeur dans une lumière de la plaque d'ostéosynthèse vertébrale selon l'invention à deux positions d'accueil ;
- la figure 9 représente une vue en perspective de la phase de positionnement de la plaque d'ostéosynthèse vertébrale selon l'invention ; et
- la figure 10 représente une vue en perspective de la phase de fixation de la plaque d'ostéosynthèse vertébrale selon l'invention.

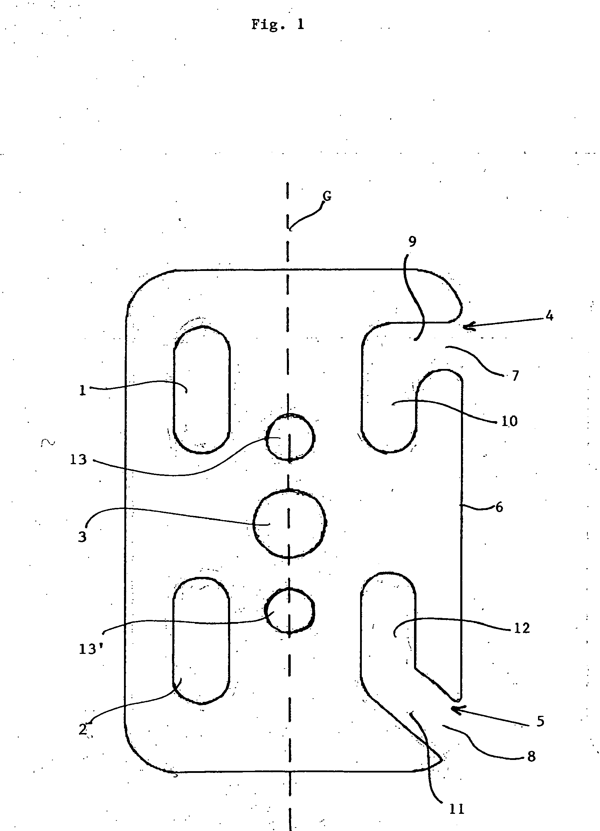

La plaque d'ostéosynthèse vertébrale selon l'exemple non limitatif décrit en référence à la figure 1 est de forme générale rectangulaire et présente quatre lumières (1, 2, 4, 5) pour le passage d'un moyen de fixation de la plaque d'ostéosynthèse vertébrale, comme par exemple une vis de fixation osseuse (20). Une lumière médiane (3) permet la fixation d'un greffon osseux ou tout autre élément de soutien intersomatique.

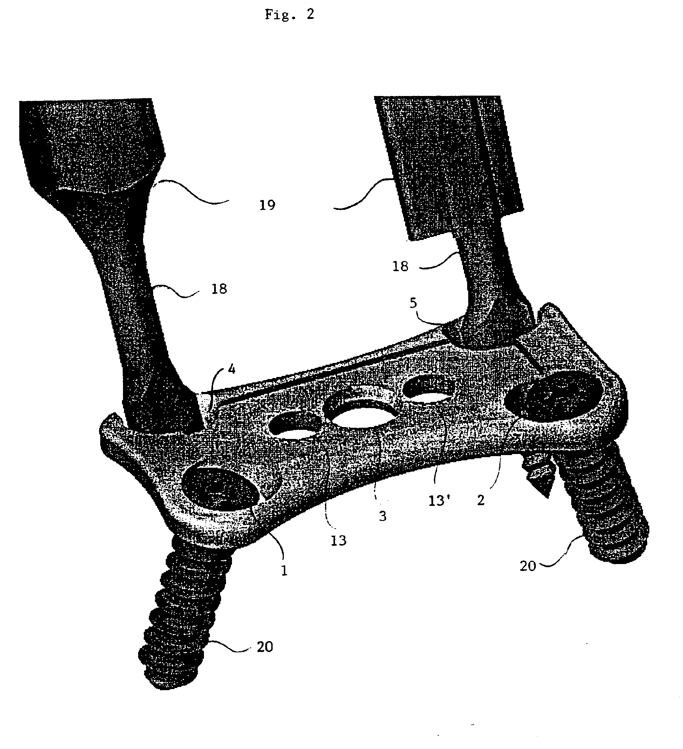

Deux lumières (4, 5) s'ouvrent sur un bord latéral (6) de la plaque pour permettre une mise en place et une fixation définitive de la plaque sans retrait préalable des tiges de correction (18) de l'instrument (19) de correction de l'orientation relative des vertèbres, comme illustré figure 2.

L'une au moins des lumières (4, 5) débouchant et de préférence les deux lumières (4, 5) débouchant présentent une forme coudée, en "L" ou autre, comprenant une partie transversale s'ouvrant sur le bord latéral (6) de la plaque par respectivement deux encoches (7, 8), un premier segment transversal respectivement (9, 11) s'étendant selon une direction transversale, perpendiculaire au bord latéral (6), et un deuxième segment longitudinal respectivement (10, 12) s'étendant selon une direction sensiblement longitudinale G.

Les lumières (1, 2) sont par ailleurs oblongues.

Le pas de vis permettant la fixation des tiges de correction (18) de l'instrument (19) de correction dans les vertèbres est bien sûr, de préférence, sensiblement identique à celui des vis de fixation (20).

De préférence, la plaque selon l'invention comporte en outre au moins un trou de fixation (13,13') d'une plaque de couverture (14), comme illustré figures 1 à 3.

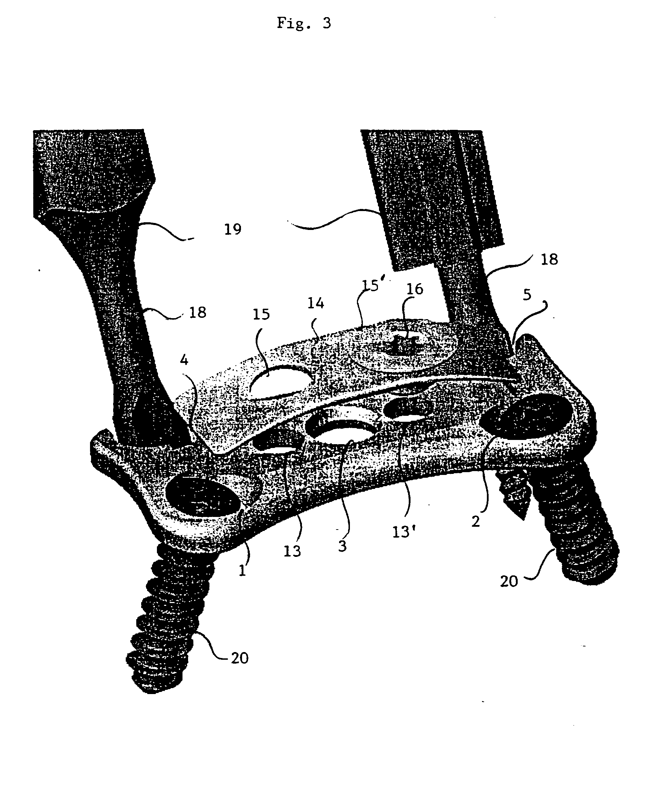

La plaque de couverture (14) comporte au moins un trou de fixation (15,15') destinés à coopérer respectivement avec les trous de fixation (13,13') afin de permettre l'introduction des vis (16,16') de fixation de la plaque de couverture (14) sur la plaque d'ostéosynthèse vertébrale. (La vis (16') n'a pas été représentée sur la figure 3 afin de maintenir la représentation suffisamment claire et précise).

La plaque de couverture (14) est destinée à venir recouvrir l'ensemble des vis de fixation. La plaque de couverture (14) présente en coupe longitudinale une forme cintrée afin d'empêcher le desserrage des vis (16,16'), par effet de ressort et afin de l'adapter au cintrage de la plaque d'ostéosynthèse. Pour améliorer ce dernier effet, au moins un des trous de fixation (15,15') est en oblong.

La plaque de couverture (14) empêche le desserrage des vis (16,16') par effet ressort, mais ne bloque pas complètement les vis de fixation (20), car ces dernières peuvent toujours bouger dans les deuxièmes segments longitudinaux des lumières (1, 2, 4, 5), afin de permettre de compenser automatiquement les effets du tassement de l'espace intervertébral.

De préférence, la plaque de couverture (14) est positionnée dans un logement ménagé à la surface de la plaque d'ostéosynthèse.

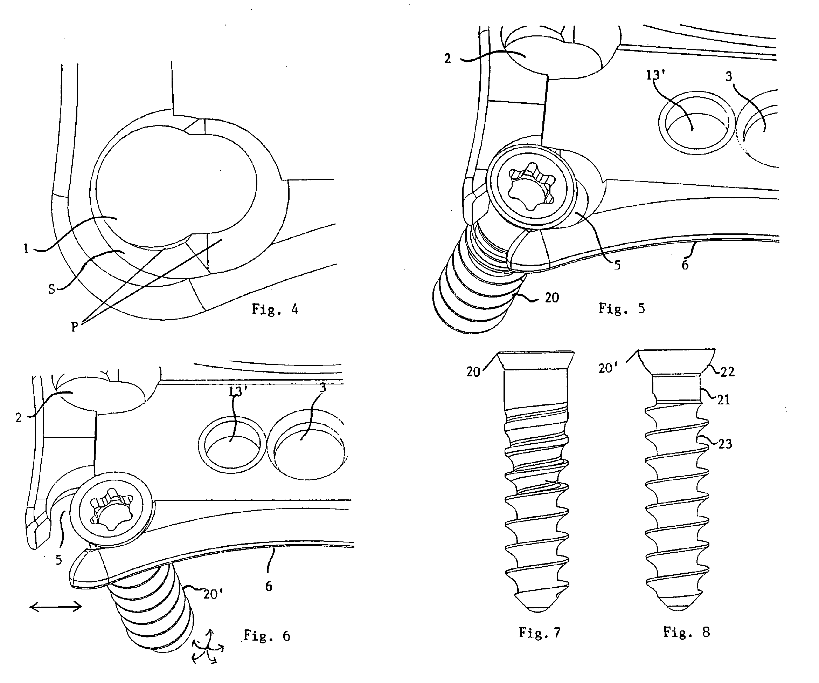

Selon un mode de réalisation particulier, l'une au moins desdites lumières (1, 2, 4, 5) pour le passage d'un moyen de fixation, comme illustré figure 4, présente au moins deux positions d'accueil S et P d'un moyen de fixation : une position S d'accueil en surface dans laquelle ledit moyen de fixation est sensiblement oblique selon un angle par rapport à ladite plaque, fixe, comme illustré figure 5 et une position P d'accueil en profondeur dans laquelle ledit moyen de fixation présente une position oblique selon un angle variable, comme illustré figure 6.

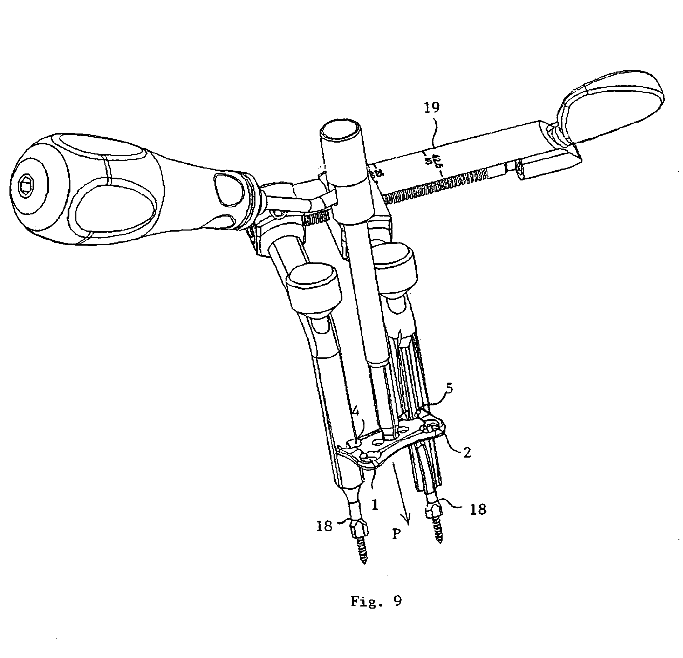

Pour un serrage à angle fixe des moyens de fixation dans la position d'accueil en surface, il convient de choisir une vis de fixation osseuse (20), traditionnelle, comme illustré figure 7, alors que pour un serrage à angle variable autorisant également la translation des moyens de fixation dans la position d'accueil en profondeur, il convient de choisir une vis de fixation osseuse (20') présentant une tête (22) dont la face inférieure est arrondie et présentant une partie à section restreinte (21) positionnée entre la tête (22) et la partie comprenant le pas de vis (23), comme illustré figure 8.

L'utilisation de la plaque selon l'invention est la suivante :

On équipe les vertèbres avec des tiges de correction (18) afin de permettre le réalignement des vertèbres avec un instrument de correction (19) et on enlève le disque intervertébral.

Il peut alors être procédé à l'introduction du greffon osseux, puis, éventuellement à la compression du greffon grâce à l'instrument de correction.

Les vis ou tiges mises en place pour le réalignement restent en position, et la plaque est positionnée suivant la flèche de positionnement P, comme illustré figure 9, en enfilant les lumières (4, 5) sur les tiges de correction (18). La plaque est alors appliquée sur les vertèbres.

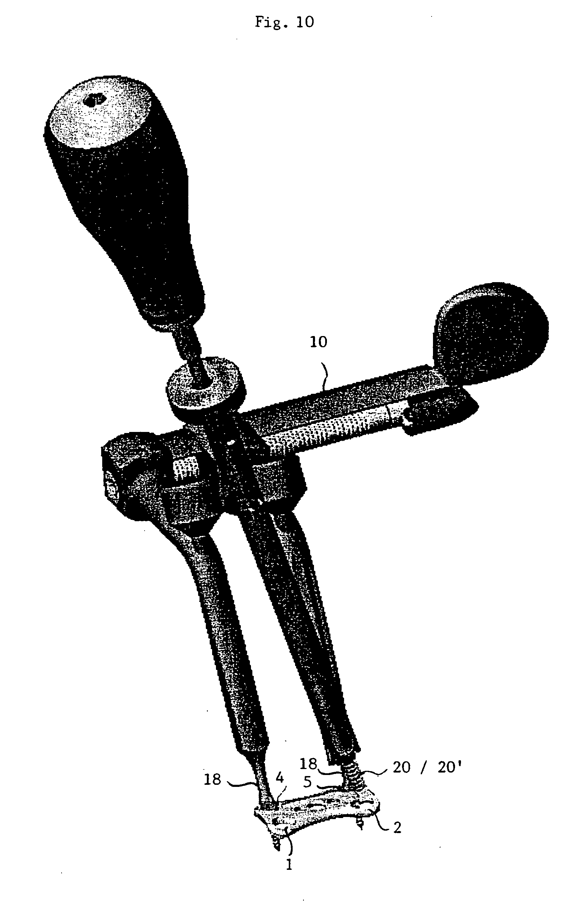

Ensuite, on procède à la fixation de la plaque d'ostéosynthèse vertébrale avec des vis de fixation osseuses (20 ou 20'), introduite dans les lumières (1, 2), comme illustré figure 10. Pendant cette opération, l'alignement des vertèbres est maintenu par les tiges de correction (18) et permet d'éviter tout risque de relâchement de la contrainte exercée sur le greffon osseux.

L'instrument de correction (19) peut alors être retiré des lumières latérales (4, 5).

Des vis de fixation osseuses (20 ou 20') peuvent alors être positionnées dans les lumières (4, 5), afin de parfaire la fixation de la plaque d'ostéosynthèse vertébrale.

Ensuite, la plaque de couverture (14) est fixée sur la plaque d'ostéosynthèse vertébrale.

## Revendications

1. Plaque d'ostéosynthèse vertébrale pour le maintien de la correction de l'orientation relative des vertèbres à l'aide d'un instrument (19) de correction, ladite plaque d'ostéosynthèse étant de forme générale rectangulaire et présentant au moins quatre lumières (1, 2, 4, 5) pour le passage, dans chacune, d'un moyen de fixation, **caractérisée en ce qu'**au moins deux lumières (4, 5) s'ouvrent sur un bord latéral (6) de ladite plaque d'ostéosynthèse pour permettre une mise en place et une fixation de la plaque sans retrait préalable de l'instrument (19) de correction de l'orientation relative des vertèbres, le bord de la plaque (6) étant défini par un bord situé dans la même direction que la direction longitudinale de la plaque, la direction longitudinale de la plaque étant sensiblement confondue avec la direction de la colonne vertébrale.

2. Plaque d'ostéosynthèse vertébrale selon la revendication 1, **caractérisée en ce que** l'une au moins des lumières (4, 5) débouchant sur le bord latéral (6) de la plaque présente une forme coudée, en "L" ou autre, comprenant un premier segment transversal s'ouvrant sur le bord latéral (6) de la plaque prolongé par un deuxième segment longitudinal orienté selon une direction sensiblement longitudinale.

3. Plaque d'ostéosynthèse vertébrale selon la revendication 1 ou 2, **caractérisée en ce que** l'une au moins des lumières (1, 2) présente un deuxième segment longitudinal orienté selon une direction sensiblement longitudinale, afin de permettre la compensation post-opératoire automatique des effets du tassement de l'espace intervertébral.

4. Plaque d'ostéosynthèse vertébrale selon l'une quelconque des revendications 1 à 3, **caractérisée en ce qu'**elle comporte en outre au moins une lumière (3) prévue sensiblement sur l'axe longitudinal médian.

5. Plaque d'ostéosynthèse vertébrale selon l'une au moins des revendications précédentes, **caractérisée en ce que** l'espacement des lumières (4, 5) débouchant sur le bord latéral (6) de la plaque correspond à la distance intersomatique entre N vertèbres, N étant un nombre entier supérieur à deux.

6. Plaque d'ostéosynthèse vertébrale selon l'une au moins des revendications précédentes, **caractérisée en ce** l'une au moins desdites lumières (1, 2, 4, 5) pour le passage d'un moyen de fixation, présente au moins deux positions d'accueil d'un moyen de fixation : une position S d'accueil en surface dans laquelle ledit moyen de fixation est sensiblement oblique selon un angle fixe par rapport à ladite plaque, et une position P d'accueil en profondeur dans laquelle ledit moyen de fixation présente une position oblique selon un angle variable.

7. Plaque d'ostéosynthèse vertébrale selon l'une au moins des revendications précédentes, **caractérisée en ce qu'**elle comporte en outre au moins un trou de fixation (13,13') d'une plaque de couverture (14) destinée à couvrir au moins partiellement lesdits moyens de fixation, après fixation.

8. Plaque d'ostéosynthèse vertébrale selon la revendication 7, **caractérisée en ce que** ladite plaque de couverture (14) est cintrée.

9. Plaque d'ostéosynthèse vertébrale selon la revendication 7 ou la revendication 8, **caractérisée en ce que** ladite plaque de couverture (14) comporte au moins un trou de fixation (15,15').

10. Plaque d'ostéosynthèse vertébrale selon la revendication 9, **caractérisée en ce qu'**au moins un trou de fixation (15,15') est oblongue.

11. Système d'ostéosynthèse vertébrale comprenant une pluralité de plaques d'ostéosynthèse vertébrale pour le maintien de la correction de l'orientation relative des vertèbres réalisée à l'aide d'un instrument (19) de correction, lesdites plaques d'ostéosynthèse étant de forme générale rectangulaire et présentant chacune au moins quatre lumières (1, 2, 4, 5) pour le passage, dans chacune des lumières, d'un moyen de fixation, au moins deux lumières (4, 5) s'ouvrant sur un bord latéral (6) de ladite plaque d'ostéosynthèse pour permettre une mise en place et une fixation de la plaque sans retrait préalable de l'instrument (19) de correction de l'orientation relative des vertèbres, l'espacement des lumières étant spécifique à chaque plaque pour permettre la sélection par le chirurgien de la plaque présentant un espacement des lumières proche de la distance intervertébrale des vertèbres du patient en cours d'opération et le bord de la plaque (6) étant défini par un bord situé dans la même direction que la direction longitudinale de la plaque, la direction longitudinale de la plaque étant sensiblement confondue avec la direction de la colonne vertébrale.

12. Système d'osthéosynthèse selon la revendication 11, **caractérisé en ce qu'**il comporte au moins une vis de fixation osseuse (20') présentant une tête (22) et une partie à section restreinte (21).

13. Système d'osthéosynthèse selon la revendication 12, **caractérisé en ce que** ladite partie à section restreinte (21) est située entre la tête (22) et la partie comprenant le pas de vis (23).

## Claims

1. Vertebral osteosynthesis plate for maintaining the correction of the relative orientation of the vertebrae by means of a correction instrument (19), said osteosynthesis plate being generally rectangular in shape and having at least four openings (1, 2, 4, 5) for fixing means to pass through each of the openings, **characterised in that** at least two of the openings (4, 5) open onto a lateral edge (6) of said osteosynthesis plate so as to allow the plate to be installed and fixed without the prior removal of the instrument (19) for correcting the relative orientation of the vertebrae, the edge of the plate (6) being defined by an edge running in the same direction as the longitudinal direction of the plate, the longitudinal direction of the plate being approximately the same as the direction of the vertebral column.

2. Vertebral osteosynthesis plate according to claim 1, **characterised in that** at least one of the openings (4, 5) that open onto the lateral edge (6) of the plate is bent at an angle, in the shape of an L or any other shape, comprising a first transversal segment that opens onto the lateral edge (6) of the plate and extended by a second longitudinal segment running in an approximately longitudinal direction.

3. Vertebral osteosynthesis plate according to claim 1 or 2, **characterised in that** at least one of the openings (1, 2) has a second longitudinal segment running in an approximately longitudinal direction, in order to enable automatic post-operation compensation of the effects of compressing the intervertabral space.

4. Vertebral osteosynthesis plate according to any one of the claims from 1 to 3, **characterised in that** it also comprises at least one opening (3) made approximately on the median longitudinal axis.

5. Vertebral osteosynthesis plate according to at least one of the preceding claims, **characterised in that** the spacing of the openings (4, 5) that open onto the lateral edge (6) of the plate corresponds to the intersomatic distance between N vertebrae, where N is a whole number that is higher than two.

6. Vertebral osteosynthesis plate according to at least one of the preceding claims, **characterised in that** at least one of said openings (1, 2, 4, 5) for the passage of fixing means has at least two positions for receiving fixing means: a surface reception position S in which said fixing means are approximately oblique at a fixed angle in relation to said plate, and a deep reception position P in which said fixing means are in an oblique position at a variable angle.

7. Vertebral osteosynthesis plate according to at least one of the preceding claims, **characterised in that** it also comprises at least one fixing hole (13, 13') for a cover plate (14) intended for covering, at least partially, said fixing means after fixing.

8. Vertebral osteosynthesis plate according to claim 7, **characterised in that** said cover plate (14) is waisted.

9. Vertebral osteosynthesis plate according to claim 7 or claim 8, **characterised in that** said cover plate (14) comprises at least one fixing hole (15, 15').

10. Vertebral osteosynthesis plate according to claim 9, **characterised in that** at least one of the fixing holes (15, 15') is oblong.

11. Vertebral osteosynthesis system comprising a multiplicity of vertebral osteosynthesis plates to maintain the correction of the relative orientation of the vertebrae provided by means of a correction instrument (19), said osteosynthesis plates being generally rectangular in shape and each having at least four openings (1, 2, 4, 5) for fixing means to pass through each of the openings, at least two of the openings (4, 5) opening on a lateral edge (6) of said osteosynthesis plate so as to allow the plate to be installed and fixed without the prior removal of the instrument (19) for correcting the relative orientation of the vertebrae, the spacing of the openings being specific to each plate so as to allow the surgeon to select a plate with its openings spaced as close as possible to the intervertebral distance of the vertebrae of the patient being operated on, and the edge of the plate (6) being defined by an edge running in the same direction as the longitudinal direction of the plate, the longitudinal direction of the plate being approximately the same as the direction of the vertebral column.

12. Osteosynthesis system according to claim 11, **characterised in that** it comprises at least one bone-fixing screw (20') with a head (22) and a part with a smaller cross-section (21).

13. Osteosynthesis system according to claim 12, **characterised in that** said part with a smaller cross-section (21) is located between the head (22) and the part comprising the screw thread (23).

## Patentansprüche

1. Platte zur Knochenvereinigung der Wirbel für die Aufrechterhaltung der Korrektur der relativen Orientierung der Wirbel mithilfe eines Korrekturinstruments (19), wobei die genannte Platte zur Knochenvereinigung eine allgemeine rechteckige Form aufweist und wenigstens vier Öffnungen (1, 2, 4, 5) für den jeweiligen Durchgang eines Befestigungsmittels aufweist, **dadurch gekennzeichnet, dass** wenigstens zwei Öffnungen (4, 5) in einen seitlichen Rand (6) der genannten Platte zur Knochenvereinigung münden, um ein Einsetzen und eine Befestigung der Platte ohne vorherige Abnahme des Korrekturinstruments (19) der relativen Ausrichtung der Wirbel zu ermöglichen, wobei der Rand der Platte (6) durch einen sich in derselben Richtung wie die Längsrichtung der Platte befindenden Rand definiert wird, wobei die Längsrichtung der Platte deutlich mit der Richtung der Wirbelsäule zusammenfällt.

2. Platte zur Knochenvereinigung gemäß Anspruch 1, **dadurch gekennzeichnet, dass** wenigstens eine der in den seitlichen Rand (6) der Platte mündenden Öffnungen (4, 5) eine gewinkelte L- oder andere Form aufweist, die ein in den lateralen Rand (6) der durch ein zweites, gemäß einer deutlichen Längsrichtung ausgerichtetes Längssegment verlängerten Platte mündendes transversales erstes Segment umfasst.

3. Platte zur Knochenvereinigung der Wirbel gemäß Anspruch 1 oder 2, **dadurch gekennzeichnet, dass** wenigstens eine der Öffnungen (1, 2) ein zweites, gemäß einer deutlichen Längsrichtung ausgerichtetes Längssegment aufweist, um die automatische Kompensation der Wirkungen des Zusammendrückens des Zwischenraums zwischen den Wirbeln nach der Operation zu ermöglichen.

4. Platte zur Knochenvereinigung der Wirbel gemäß Anspruch 1 bis 3, **dadurch gekennzeichnet, dass** sie darüber hinaus wenigstens eine deutlich auf der medianen Längsachse vorgesehene Öffnung (3) umfasst.

5. Platte zur Knochenvereinigung der Wirbel gemäß einem der vorherigen Ansprüche, **dadurch gekennzeichnet, dass** die Beabstandung der in den seitlichen Rand (6) der Platte mündenden Öffnungen (4, 5) dem intervertebralen Abstand zwischen N Wirbeln entspricht, wobei N eine ganze Zahl größer als zwei ist.

6. Platte zur Knochenvereinigung der Wirbel gemäß wenigstens einem der vorherigen Ansprüche, **dadurch gekennzeichnet, dass** wenigstens eine der genannten Öffnungen (1, 2, 4, 5) für den Durchgang eines Befestigungsmittels wenigstens zwei Aufnahmepositionen eines Befestigungsmittels aufweist: Eine Aufnahmeposition S an der Oberfläche, in der das genannte Befestigungsmittel gemäß einem festen Winkel im Verhältnis zur genannten Platte deutlich geneigt ist, und eine Aufnahmeposition P in der Tiefe, in der das genannte Befestigungsmittel eine gemäß einem variablen Winkel geneigte Position aufweist.

7. Platte zur Knochenvereinigung der Wirbel gemäß wenigstens einem der vorherigen Ansprüche, **dadurch gekennzeichnet, dass** sie darüber hinaus wenigstens ein Befestigungsloch (13, 13') einer zum wenigstens teilweisen Abdecken der genannten Befestigungsmittel nach der Befestigung bestimmte Deckplatte (14) umfasst.

8. Platte zur Knochenvereinigung der Wirbel gemäß Anspruch 7, **dadurch gekennzeichnet, dass** die genannte Deckplatte (14) gewölbt ist.

9. Platte zur Knochenvereinigung der Wirbel gemäß Anspruch 7 oder Anspruch 8, **dadurch gekennzeichnet, dass** die genannte Deckplatte (14) wenigstens ein Befestigungsloch (15, 15') umfasst.

10. Platte zur Knochenvereinigung der Wirbel gemäß Anspruch 9, **dadurch gekennzeichnet, dass** wenigstens ein Befestigungsloch (15, 15') länglich ist.

11. System zur Knochenvereinigung der Wirbel mit einer Vielzahl von Platten zur Knochenvereinigung der Wirbel für die Aufrechterhaltung der mithilfe eines Korrekturinstruments (19) realisierten Korrektur der relativen Ausrichtung der Wirbel, wobei die genannten Platten zur Knochenvereinigung eine allgemeine rechteckige Form aufweisen und jede wenigstens vier Öffnungen (1, 2, 4, 5) für den Durchgang eines Befestigungsmittels durch jede Öffnung aufweist, wobei wenigstens zwei Öffnungen (4, 5) in einen seitlichen Rand (6) der genannten Platte zur Knochenvereinigung münden, um ein Einsetzen und eine Befestigung der Platte ohne vorheriges Abnehmen des Korrekturinstruments (19) der relativen Ausrichtung der Wirbel zu ermöglichen, wobei die Beabstandung der Öffnungen für jede Platte spezifisch ist, um die Auswahl der eine der intervertebralen Entfernung der Wirbel des sich in der Operationen befindenden Patienten ähnlichen Beabstandung der Löcher aufweisende Platte durch den Chirurgen zu erlauben, und wobei der Rand der Platte (6) durch einen sich in derselben Richtung wie die Längsrichtung der Platte befindenden Rand definiert ist, wobei die Längsrichtung der Platte deutlich mit der Richtung der Wirbelsäule zusammenfällt.

12. System zur Knochenvereinigung gemäß Anspruch 11, **dadurch gekennzeichnet, dass** es wenigstens eine einen Kopf (22) und einen Teil mit verkleinertem Durchmesser (21) aufweisende Befestigungsschraube (20') am Knochen umfasst.

13. System zur Knochenvereinigung gemäß Anspruch 12, **dadurch gekennzeichnet, dass** der genannte Teil mit verkleinertem Durchmesser (21) sich zwischen dem Kopf (22) und dem den Gewindeschritt (23) umfassenden Teil befindet.
